# EUROPEAN PATENT APPLICATION

(11) **EP 4 374 793 A1**
(43) Date of publication of application: **29.05.2024**
(21) Application number: 23207458.3
(22) Date of filing: 02.11.2023
(51) Int. Cl.: A61B 10/00, A61B 5/157, A61B 5/15

(54) **DEVICE FOR TESTING ANALYTE IN LIQUID SAMPLE**

(30) Priority: 23.11.2022 CN 202211478145; 28.11.2022 US 202263428226 P
(71) Applicant: Hangzhou Biotest Biotech Co., Ltd., Yuhang District Hangzhou (CN)
(72) Inventor: WU, Shujiang, Hangzhou (CN); HONG, Liang, Hangzhou (CN)
(74) Representative: Piotrowicz, Pawel Jan Andrzej

(57) **Abstract**

The present invention provides a device for testing an analyte in a liquid sample, and the device includes a detection chamber, where the detection chamber is configured to receive a testing element, and the testing element is configured to test the analyte in the liquid sample; and a sampling element, where the sampling element is configured to absorb the liquid sample. The test device can be used for self-test of infectious diseases including AIDS.

## Description

### CROSS-REFERENCE TO RELATED APPLICATIONS

The present invention claims priority to the Chinese Patent Application, Application No. 202211478145.3, filed on November 23, 2022, and to the U.S. Provisional Application, Application No. US63/428,226, filed on November 28, 2022, and all disclosures of the Applications, including claims, abstract, specification, and accompanying drawings of the specification, are hereby incorporated by reference in their entirety as a part of the present invention.

### BACKGROUND OF THE INVENTION

### Field of the Invention

The present invention relates to a device for collecting a liquid sample, in particular, a device for collecting and testing an analyte in a liquid sample in the field of rapid diagnosis, such as a urine and saliva collection and test device.

### Description of the Related Art

The following description is merely an introduction of some background knowledge and does not constitute any limitation to the present invention.

At present, the test device for detecting the presence or absence of an analyte in sample is widely used in hospitals or homes, and such test device for rapid diagnosis includes one or more test strips, such as early pregnancy detection and drug abuse detection. Such test device for rapid diagnosis is very convenient, and can obtain a testing result from the test strips after one minute or no at most about ten minutes.

Drug test is widely used by the drug control department, the Public Security Bureau, drug rehabilitation centers, physical examination centers, physical examination offices of national conscription, etc. The drug test is diverse and frequent. Some detections are required to collect samples and then samples are detected in professional testing agencies or testing laboratories, and some detections need to be completed in the site in time, for example, persons who drive after drug use need to be tested on the spot (referred to as "Drug Driving"), to obtain the testing results in time.

For example, the detection of saliva samples is gradually accepted and favored by testing agencies or testing personnel due to convenient collection. In some literatures, various sample collection and test devices for clinical and domestic uses have been described. For example, the US Patent No. 5,376,337 discloses a saliva sampling device in which a piece of filter paper is used to collect saliva from the mouth of a subject and deliver saliva to an indicator reagent. US patents US 5,576,009 and US 5,352,410 each disclose a syringe-type liquid sampling device.

In addition, family self-test is increasingly common, and most self-test products available for COVID-19. Infectious diseases are becoming increasingly popular at home, and it is necessary to design the existing products, so that these products can realize family self-test.

In view of the above technical problems in some conventional products, it is necessary to improve them and provide an alternative approach to solve the drawbacks of the prior art, especially for an infectious disease self-test device or method.

### BRIEF SUMMARY OF THE INVENTION

In order to overcome some drawbacks in conventional technologies, the present invention provides a self-test device. The device can be used to self-test an analyte in a blood sample at home, and features operation convenience, safety and reliability.

Therefore, on the one hand, the present invention provides a device for testing an analyte in a liquid sample, and the device includes a detection chamber, where the detection chamber is configured to receive a testing element, and the testing element is configured to test the analyte in the liquid sample; and a sampling element, where the sampling element is configured to absorb the liquid sample. Such absorption is automatic absorption, and the liquid sample is automatically absorbed under capillary action. In some embodiments, the sampling element and the detection chamber are integrally designed. In some embodiments, the sampling element includes one or more capillary channels, and the liquid sample is absorbed by the capillary action of the capillary channel. In some embodiments, the sampling element includes a capillary channel for automatically absorbing the liquid sample.

In some embodiments, the capillary channel is in fluid communication with the detection chamber. In some embodiments, the liquid sample automatically absorbed by the capillary channel is located in the capillary channel and cannot automatically flow into the detection chamber. In some embodiments, the sampling element is the capillary channel, and the capillary channel is capable to automatically absorb the liquid sample and is in fluid communication with the detection chamber. A capillary channel structure herein includes individual capillary channels, and these capillary channels can automatically absorb the liquid sample therein according to surface tension. However, these samples in the capillary channel will not automatically flow into the detection chamber. This is the ability of the capillary channel itself. When the capillary channel automatically absorbs the liquid sample, the liquid sample will be automatically retained in the capillary channel, instead of automatically flowing out of the capillary channel. In some embodiments, the capillary channel is provided in one or plurality, and each capillary channel is capable to automatically absorb the liquid sample.

In some embodiments, the sampling element includes a capillary channel, where one end of the capillary channel is in fluid communication with the detection chamber and the other end thereof is in communication with the outside. For example, the capillary channel includes an inlet for absorbing the liquid sample and an outlet for flowing out of the liquid sample, the inlet is in communication with the outside, and the outlet is in fluid communication with the detection chamber. In some embodiments, the capillary channel structure is provided in the sampling element; the sampling element has an insertion end; the insertion end is provided to be inserted into a chamber; and the chamber may include the treatment liquid or may not include any liquid. In some embodiments, the inlet is located in or on the insertion end, or the inlet separately extends outward from an end portion of the insertion end.

In the absence of the treatment liquid, the sampling element forms a sealing chamber in a container when being inserted. With the sampling element being inserted, gas in the sealing chamber is compressed, increased pressure allows a part of the gas to enter the capillary channel. The gas entering the capillary channel causes the liquid sample staying in the capillary channel to flow into the detection chamber and contact the testing element in the detection chamber to complete test.

In some embodiments, a chamber accommodating the treatment liquid is provided in a container. When the sampling element is inserted into the chamber accommodating the treatment liquid, the sampling element forms a sealing chamber in the chamber accommodating the treatment liquid; the sealing chamber includes the treatment liquid therein; with the sampling element being inserted, the pressure of the sealing chamber increases, the increased pressure causes a part of the treatment liquid to flow into the capillary channel, thereby facilitating the liquid sample in the capillary channel and an effluent liquid to together enter the detection chamber.

In some embodiments, the sampling end being inserted causes the treatment liquid to flow into the capillary channel, thereby causing the treatment liquid and the liquid sample to together enter the detection chamber and contact the testing element in the detection chamber. In other words, the liquid sample in the capillary channel needs to flow into the capillary channel under
external pressure, such that the liquid sample can flow out of the capillary channel and enter the detection chamber. Therefore, when the capillary channel is used to absorb the liquid sample, the absorbed liquid sample is located in the capillary channel and cannot automatically flow into the detection chamber, such that sampling and detection can be performed step by step. For example, the sampling element with the testing element is used to absorb the liquid sample. For example, a fingertip blood directly contacts one end of the capillary channel (for example, inlet). A part of the blood samples enters the capillary channel under the capillary action thereof, but the blood sample therein is retained in the capillary channel, instead of flowing into the detection chamber. The sampling element is inserted into the chamber of the container if required to be operated. The sealing chamber is formed in the chamber; with the sampling element being inserted, the pressure of the sealing chamber increases, and the gas and the liquid sample in the chamber may enter the capillary channel; alternatively, the gas enters the capillary channel earlier than the liquid sample, such that the liquid sample staying or being preserved in the capillary channel enters the detection chamber and contacts the testing element in the detection chamber, thereby assaying the analyte in the liquid sample. The liquid herein is mainly the treatment liquid.

Therefore, the sampling element of the present invention has two functions, one function thereof is to have the capillary channel for automatically absorbing the liquid sample, such as blood, saliva, or any other liquid sample, thereby allowing the sample to be preserved in the capillary channel; the other function thereof is to allow the sampling element to fit with one chamber, thereby allowing the sampling element to be inserted to form the sealing chamber; and the continued insertion of the sampling end causes the pressure to be generated in the capillary channel. The pressure facilitates the liquid sample in the capillary channel to flow out of the capillary channel and enter the detection chamber. It can be understood that a sample end is inserted into the chamber, and one end of the capillary channel that is in communication with the outside is in fluid communication with the chamber, such that the pressure change of the sealing chamber is directly reflected in the capillary channel. For example, the gas is compressed, air pressure increases and a part of gas enters the capillary channel, thereby facilitating the liquid sample retained in the capillary channel to flow out of the capillary channel, for example, to flow into the detection chamber.

Therefore, in some embodiments, the sampling element has an insertion end that is provided with the inlet of the capillary channel; the sample enters from the inlet to the capillary channel and stays in the capillary channel when being sampled; the present invention further provides a chamber having an opening; the chamber allows the sampling element to be inserted through the opening; when the insertion end of the sampling element is inserted, the opening is sealed, and a hermetic sealing space is formed in the chamber; when the sampling element continues to be inserted into the chamber, the gas in the sealing space is compressed and the air pressure increases, and pressure is generated at the inlet of the capillary channel; such pressure causes the liquid sample in the capillary channel to flow into the detection chamber; and actually, a part of the gas enters from the inlet, thereby facilitating the liquid sample staying in the capillary channel to flow out of the capillary channel. Of course, pressure herein can be generated by such a way that the insertion end of the sampling element being inserted into the chamber allows air in the chamber to be compressed; alternatively, the solution chamber has the liquid sample, the insertion end is inserted into the chamber to apply pressure to the liquid sample, and the pressure allows the treatment liquid to flow into the capillary channel, thereby allowing the liquid sample in the capillary channel to flow into the detection chamber; and of course, the treatment liquid and the sample together enter the detection chamber.

In some embodiments, in order to allow the insertion end of the sampling element in all or in part to be inserted into the chamber, pressure can be generated. When the insertion end is inserted into the chamber, the sealing space is generated between the insertion end and the chamber and needs to be generated at least temporarily; therefore, when the insertion end is inserted, the internal air or internal liquid can be compressed temporarily, thereby generating pressure or increasing the internal pressure. The capillary channel is located inside the insertion end, such that the increased pressure will enter the capillary channel to cause the liquid sample therein to flow into the detection chamber. In some embodiments, the shape of the insertion end matches the inner shape of the container. Therefore, when the insertion end is inserted into the container, only a narrow space is left between an outer portion of the insertion end and an inner wall of the container. Matching used herein means that with the insertion end being inserted into the container, only more than 60% of the space is occupied by the insertion end, for example, 65%, 70%, 75%, 80%, 85%, 90%, 95%, 96%, 97%, 98% and 99%. The short time of pressure herein can be several seconds, such as 1 seconds, 2 seconds, 3 seconds, 5 seconds, 10 seconds, and 15 seconds. This is the whole process where the insertion end is inserted into the chamber.

In some embodiments, the shape of the sampling element matches with the shape of the space in the container where the sampling element is inserted, such that the sampling element can be inserted into the inserted container. Although the sampling end does not seal the container, pressure will also be generated in a short time, such that the gas or liquid in the inserted container can enter the capillary channel, thereby causing the liquid sample therein to flow out of the capillary channel. Matching used herein means that the insertion element or the whole sampling element completely and substantially fills the whole chamber when being inserted into the chamber. If the chamber includes liquid therein, a part of the liquid flows into the capillary channel, and the part of the treatment liquid flowing into the capillary channel facilitates the liquid sample therein to flow out of the capillary channel and flow into the detection chamber to contact the testing element.

In some embodiments, the sampling end has a narrow end and a relatively wide end, where the narrow end is far away from the detection chamber, the relatively wide end is proximal to the detection chamber, and the narrow end has the inlet of the capillary channel thereon. This is a cone-like design. The container into which the sampling end is inserted is also conical in design. The container can only include gas therein, and an optimal way thereof is to include liquid. When the insertion element is inserted into the container and fits with the container, the sampling end occupies the space of the container in all or almost completely, and only a narrow space is formed between the insertion element and the sampling end. Therefore, enough pressure can be generated in a short insertion process and can also allow the treatment liquid to flow into the capillary channel through the inlet thereof, thereby facilitating the liquid sample therein to flow out of the capillary channel through the outlet thereof and flow into the detection chamber. In some embodiments, the inlet of the capillary channel is not sealed during insertion. Therefore, when enough pressure is generated, such pressure causes the liquid to flow into the capillary channel, thereby allowing the liquid sample therein to flow into the detection chamber. If the liquid sample flows into the capillary channel and the insertion element fits with the inserted container, a narrow space or a short distance is provided between the insertion element and the inserted container; the sampling end is inserted into the container in a short time, generally it is difficult for the liquid sample to flow out of a narrow gap, but the liquid sample is allowed to flow into the inlet of the capillary channel, and the treatment liquid flowing into the capillary channel facilitates the liquid sample therein to flow out of the capillary channel.

In some embodiments, when the insertion end is inserted, the sealing space is formed in the solution chamber. For example, in some embodiments, the solution chamber has an opening, and the insertion end has a sealing ring. When the insertion end is inserted into the opening, a sealing ring at the insertion end seals the opening, thus forming the sealing chamber in the solution chamber. With the insertion end being inserted, the pressure in the sealing space increases, the sealing space includes a solution therein, and the increased pressure causes the solution to flow into the capillary channel, thereby causing the liquid sample therein to flow into the detection chamber and contact with the testing element. In some embodiments, the insertion end is a part of a sampling element. In some embodiments, the insertion end includes the capillary channel therein. It can be understood that the solution chamber does not include solution therein, but only includes gas. Hermetic seal or liquid seal is realized by an elastic sealing ring on the insertion element or the sampling element. This is realized through the fit of the elastic sealing ring and the side walls of the chamber.

In some embodiments, when the insertion end is inserted into the container, the insertion end is locked with the container, such that the container and the insertion end are connected into an integrated structure.

In some embodiments, the shape of the solution chamber matches that of the insertion end. Therefore, the insertion end being inserted into the container chamber causes the part of the treatment solution to flow into the capillary channel, thereby allowing the liquid sample and the treatment liquid in the capillary channel to together flow into the detection chamber.

In some embodiments, a buffer chamber is further provided near the outlet of the capillary channel and in fluid communication with the detection chamber. The purpose of the buffer chamber is to allow the liquid flowing out of the capillary channel through the outlet thereof to stay in the buffer chamber, reducing the flow rate thereof. Another function thereof is that the treatment liquid is generally located behind the liquid sample in the capillary channel if treated, and the treatment liquid therein cannot be mixed with the liquid sample therein because the treatment liquid in the capillary channel occupies a part of the capillary channel and the liquid sample is in front of the treatment liquid and also occupies a part of the capillary channel. When the buffer chamber is provided, the liquid sample flows into the buffer chamber, and the treatment liquid also flows into the buffer chamber, such that the treatment liquid can be well mixed and contact with the liquid sample, and the liquid sample can be treated; for example, the liquid sample is lysed to release the analyte, including dissolving some interfering substances, changing the properties of the sample, or increasing the volume of the liquid sample, thereby allowing the mixture to contact a test strip to satisfy the required volume of the liquid sample when the test strip completes the whole flowing process. For example, the liquid sample sucked by the capillary channel is 20 µL, 200-300 µL of the liquid sample is needed if the test strip completes the whole flowing process. The treatment liquid is mixed with the liquid sample to obtain 200-300 µL of the liquid sample to complete the whole test. Of course, the buffer chamber is located between the detection chamber and the outlet of the capillary channel, thereby providing a mixing chamber for the liquid sample and the treatment liquid.

On the other hand, the present invention provides a device for testing an analyte in a liquid sample, and the device includes a detection chamber, where the detection chamber is configured to receive a testing element, and the testing element is configured to test the analyte in the liquid sample; a sampling element, where the sampling element is configured to absorb the liquid sample; and a solution chamber including a treatment liquid, configured to accommodate the treatment liquid for treating the liquid sample, where when a part of the sampling element is inserted into the solution chamber, the sampling element causes the treatment solution in the solution chamber to flow into the capillary channel.

In some embodiments, the sampling element includes a capillary channel by which the liquid sample is automatically absorbed and preserved. A sampling distance facilitates the treatment liquid to flow into the capillary channel, thereby facilitating the liquid sample to flow out of the capillary channel.

In some embodiments, the capillary channel of the sampling element is in fluid communication with the detection chamber. In some embodiments, the detection chamber includes the testing element therein.

In some embodiments, a sample application area of the testing element in the detection chamber is located near an outlet of the capillary channel.

In some embodiments, a buffer area is further provided between the outlet of the capillary channel and the sample application area of the testing element, and used for mixing the liquid sample with the treatment liquid. In some embodiments, the buffer area is a chamber, and the volume of the chamber is greater than or equal to the sum of the volumes of the liquid sample and the treatment liquid.

On the other hand, the present invention provides a method for testing an analyte in a liquid sample, and the method includes:
providing a device, where the device includes a detection chamber; the detection chamber is configured to receive a testing element, and the testing element is configured to test the analyte in the liquid sample; and a sampling element, where the sampling element is configured to collect the liquid sample, and the sampling element includes a capillary channel;
allowing the capillary channel of the sampling element to absorb the liquid sample; allowing the sample to be preserved in the capillary channel; then inserting the sampling element into a solution chamber including a treatment liquid such that the sampling element causes the treatment liquid in the solution chamber to flow into the capillary channel, whereby allowing the liquid sample in the capillary channel to flow into the detection chamber.

In some embodiments, the sampling element is inserted into the solution chamber to form a sealing space therein, and the sealing space is in communication with the capillary channel. When the sampling element continues to be inserted, the sealing space is compressed, the pressure therein increases, and the increased pressure allows the solution to flow into the capillary channel. The sampling element has a first position and a second position when being inserted into the solution chamber. When the sampling element is located at the first position, the sampling element seals the solution chamber or forms the sealing space in the solution chamber. When the sampling element is located at the second position, the sealing space is compressed, such that an initial treatment liquid flows into the capillary channel, and the treatment liquid flowing into the capillary channel causes the liquid sample to flow therein. Alternatively, the liquid sample therein is allowed to flow out of the capillary channel and flow into the detection chamber.

In some embodiments, a sealing ring is provided on the sampling element, and the solution chamber is provided with an opening; when the sampling element is allowed to be inserted into the opening of the solution chamber, the opening is hermetically sealed or liquid-sealed by the sealing ring, thereby forming a hermetic sealing space or a liquid sealing space in the solution chamber.

In some embodiments, a buffer area is provided between the outlet of the capillary channel and the detection chamber, such that the treatment liquid and the liquid sample are mixed in the buffer area. In some embodiments, the mixed liquid sample is allowed to flow into the detection chamber and contact the test strip of the detection chamber. In some embodiments, the mixed liquid sample is allowed to flow into the test strip and contact a sample application area of the testing element.

In the foregoing embodiments, the sample is a blood sample. The capillary channel can automatically absorb the blood sample.

In some embodiments, the blood sample is obtained from a blood collection tube or fingertip blood. The blood sample can be whole blood, serum or plasma. In some embodiments, the analyte is virus, bacterium, and fungus, or fragments of virus, bacterium and fungus, or the like. In some embodiments, the virus is HIV, COVID-19, influenza virus, or the like.

### Beneficial effect

With the above structure, the analyte in the liquid sample can be tested step by step, thereby realizing quantification and test control. In addition, the test device can be used for self-test of infectious diseases including AIDS.

### BRIEF DESCRIPTION OF THE DRAWINGS

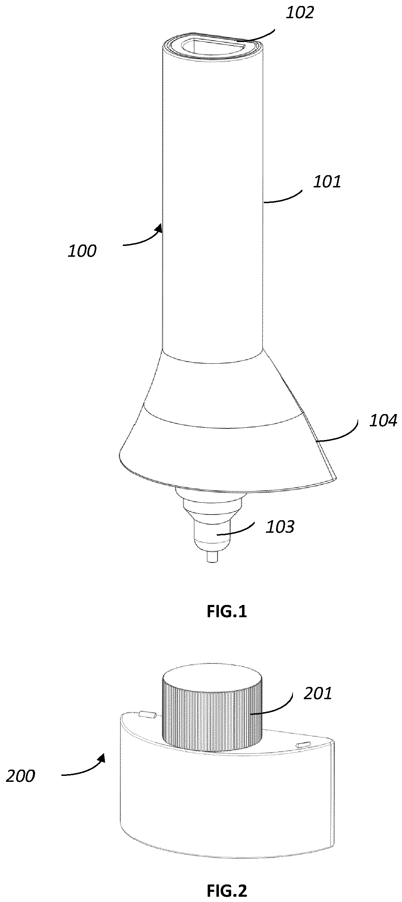

FIG. 1 is a schematic diagram showing a three-dimensional structure of a test device according to a specific embodiment of the present invention.
FIG. 2 is a schematic diagram showing a three-dimensional structure of a chamber or a solution chamber (receiving chamber) according to a specific embodiment of the present invention.
FIG. 3 is a schematic diagram showing an exploded three-dimensional structure of a detection chamber, a carrier element and a sampling element according to a specific embodiment of the present invention.
FIG. 4 is a schematic diagram showing a three-dimensional structure of a detection chamber according to a specific embodiment of the present invention (excluding a testing element and a carrier).
FIG. 5 is a schematic diagram showing a three-dimensional structure of a carrier including a testing element according to a specific embodiment of the present invention.
FIG. 6 is a schematic diagram showing a three-dimensional structure of a detection chamber according to a specific embodiment of the present invention.
FIG. 7 is a schematic diagram showing a cross-section structure of a collection element according to a specific embodiment of the present invention.
FIG. 8 is a schematic diagram showing a three-dimensional structure after a collection element and a detection chamber are assembled according to a specific embodiment of the present invention.
FIG. 9 is a schematic diagram showing a cross-section structure of a structure shown in FIG. 8.
FIG. 10 is a schematic diagram showing a cross-section structure of a solution container according to a specific embodiment of the present invention.
FIG. 11 is a schematic diagram showing a cross-section structure of a sampling element of a test device of the present invention being inserted into a solution container.

### DETAILED DESCRIPTION OF THE INVENTION

The following further describes the structures involved in the present invention or the technical terms used therein. Unless otherwise specified, they shall be understood and explained according to the general terms commonly used in the prior art.

### Detection

Detection means assaying or testing presence or absence of a substance or material, including but not limited to, chemical substance, organic compound, inorganic compound, metabolite, drug, drug metabolite, organic tissue, metabolite of organic tissue, nucleic acid, protein or polymer. In addition, detection means that the amount of a substance or material is tested. Further, assay also means immunoassay, chemical assay, enzyme assay, and the like.

### Sample

The samples detected or collected by the test device of the invention include biological liquid (for example, case liquid or clinical sample). Liquid samples or fluid samples may be derived from solid or semi-solid samples, including feces, biological tissues and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine, and preferably, the biological sample is saliva. Food samples include food processed materials, final products, meat, cheese, wine, milk, and drinking water. Plant samples include samples derived from any plants, plant tissues, plant cell cultures, and media. "Environmental samples" include samples derived from the environment (e.g., liquid samples from lakes or other bodies of water, sewage samples, earthen samples, groundwater, seawater, and waste liquid samples). The environmental sample may further include sewage or other waste water.

An appropriate testing element according to the invention can be used to detect any analyte. Preferably, the test device of the invention is used to detect small drug molecules in saliva and urine or viruses in blood samples, such as human immunodeficiency virus (HIV) and coronavirus.

### Treatment liquid

Treatment liquid is a solution or reagent used to treat a liquid sample. Different from the liquid sample, it is generally a solution prepared in advance and can elute or treat the absorption element, or treat a liquid sample, for example, adjustment of PH value, reduction of nonspecific binding, and avoidance of false positives or false negatives. The latter can improve the properties of immunoassay of an analyte in a sample on a test strip. Generally, treatment liquid does not include analyte, nor does it include components with the same properties as a sample. Therefore, when the treatment liquid is mixed with the liquid sample, a new mixed solution formed can flow to a detection chamber to contact a testing element. The treatment liquid can dissolve the sample. When the sample is solid or powder, the treatment liquid allows the sample to be dissolved and form a liquid sample.

### Downstream and upstream

Downstream or upstream is divided according to a flow direction of a liquid. Generally, a liquid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the test device of the present invention, when the fluid sample or the liquid sample is absorbed by the absorbing element, liquid can flow into the detection chamber from the liquid outlet of the capillary channel, contact with the testing element 300 thereon, and flow into the sample application area 304 of the testing element 300; in this case, the flow of the liquid in the sample application area 304 to the absorption area 303 is from upstream to downstream; in a liquid flowing process, the liquid flows from a label area 307 to a testing area 306, and a detection area and a testing result control area are provided on the testing area. The testing area may be a polyester fiber film, and the sample application area may be a glass fiber.

### Gas flow or liquid flow

Gas flow or liquid flow means that liquid or gas can flow from one place to another place. In a flow process, the liquid or gas may pass through some physical structures to play a guiding role. The "passing through some physical structures" here means that liquid passes through the surface of these physical structures or their internal space and flows to another place passively or actively, where passivity is usually caused by external forces, such as flow under the capillary action. The flow here may also be a flow due to self-action (gravity or pressure) of the liquid or gas, and also may be a passive flow. Here, the flow does not mean that a liquid or a gas is necessarily present, but indicates a relationship or state between two objects under some circumstances. In case of presence of liquid, it can flow from one object to another. Here it means the state in which two objects are connected. In contrast, if there is no gas flow or liquid flow state between two objects, and liquid exists in or above one object but cannot flow into or on another object, it is a non-flow, non-liquid or non-gas flow state.

### Detachable combination

Detachable combination means that connection relationship between two components is in several different states or positional relationship. For example, with two components being physical components, they can be separated at the beginning and then connected or combined in an appropriate first case, and separated in an appropriate second case. Physically, such separation is spatial separation without contact. Alternatively, the components are combined at the beginning, and can be physically separated from each other when appropriate. Alternatively, two objects are separated at the beginning, combined to achieve a specified function if necessary, then separated, or later combined again for a purpose. In short, combination or separation or two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

### Testing element

The "testing element" used herein refers to an element that can be used to detect whether a sample or a specimen contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the invention.

Various testing elements can be combined for use in the invention. One form of the testing elements is a test strip. The test papers used for analyzing the analyte (such as drugs or metabolites that show physical conditions) in samples can be of various forms such as immunoassay or chemical analysis. The analysis mode of non-competition law or competition law can be applied for test strips. A test strip generally contains a water absorbent material that has a sample application area, a reagent area, and a testing area. Samples are added to the sample application area and flow to the reagent area through the capillary channel. If analyte exists in the reagent area, samples will bind to the reagent. Then, samples continue to flow to the testing area. Other reagents such as molecules that specifically bind to analyte are immobilized on the testing area. These reagents react with the analyte (if any) in the sample and bind to the analyte in this area, or bind to a reagent in the reagent area. Marker used to display the detection signal exists in the reagent area or the detached label area.

Typical non-competition law analysis mode: if a sample contains analyte, a signal will be generated; and if not, no signal will be generated. Competition law: if no analyte exists in the sample, a signal will be generated; and if analyte exists, no signal will be generated.

The testing element can be a test strip, which can be water absorbent material or non-water absorbent material. The test strip can contain several materials used for delivery of liquid samples. One material of the test strip can cover the other material thereof. For example, the filter paper covers the nitrocellulose membrane. One or more materials may be used in one area of the test strip, and one or more other different materials may be used in the other area thereof. The test strip can stick to a certain support or on a hard surface for improving the strength of holding the test strip.

Analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal can be in the sample application area, the reagent area or the testing area, or on the entire test strip, and one or more materials of the test strip can be filled with this substance. The solution containing a signifier is added onto the surface of the test strip, or one or more materials of the test strip is or are immersed in a signifier-containing solution. The test strip containing the signifier solution is made dry.

Various areas of the test strip can be arranged in the following way: sample application area, reagent area, testing area, control area, an area to determine whether the sample is adulterated or not, and liquid sample absorption area. The testing result control area is located behind the testing area. All areas can be arranged on a test strip that is only made of one material. Alternatively, different areas may be made of different materials. Each area can be in direct contact with the liquid sample, or different areas are arranged according to the flow direction of liquid sample; and a tail end of each area is connected and in overlapped with the front end of the other area. Materials used can be those with good water absorption such as filter papers, glass fibers or nitrocellulose membranes. The test strip can also be in other forms.

The nitrocellulose membrane test strip is commonly used, that is, the testing area includes a nitrocellulose membrane on which a specific binding molecule is immobilized to display the testing result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or test device of the present invention for the detection of an analyte, for example, the detection of an analyte in a sample.

Test strips used in the present invention may be commonly referred as lateral flow test strips. The specific structure and detection principle of the test strips are well known to a person skilled in the art in the prior art. A common test strip includes a sample collection area or a sample application area 301, a label area 302, and a testing area 303; the sample collection area includes a sample receiving pad, the label area includes a label pad, and a water absorption area 304 may include a water absorbent pad, where the testing area includes necessary chemical substances for detecting the presence or absence of analyte, such as immunoreagents or enzyme chemical reagents. The nitrocellulose membrane test strip is commonly used, that is, the testing area includes a nitrocellulose membrane, and an area on which a specific binding molecule is immobilized to display the testing result; and other test strips such as cellulose acetate membrane or nylon membrane test strips can also be used. Of course, in the downstream of the testing area, there may also be a testing result control area; generally, test strips appear on the testing result control area and the testing area in the form of a horizontal line, namely, a test line or a control line. Such test strips are conventional. Of course, they can also be other types of test strips for detection under the capillary action. In addition, there are dry chemical reagent components on common test strips, for example, an immobilized antibody or other reagents. When the test strip contacts liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the test device of the present invention or can be disposed in contact with the liquid samples in a detection chamber or used to detect the presence or absence of analyte in the liquid samples that enter a detection chamber, or the quantity thereof. Generally, the testing element is configured in the detection chamber 105. When a liquid sample exists in the detection chamber, it contacts the testing element for assaying or testing.

### Analyte

Examples that can use an analyte related to the invention include some small-molecule substances, including drugs (such as drug of abuse). "Drug of Abuse" (DOA) refers to using a drug (playing a role of paralyzing the nerves usually) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the pre invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the present invention.

### Liquid flow

Typically, liquid flow refers to flow from one place to another place. Under normal circumstances, most of liquid flow in nature is from a high place to a low place under the action of gravity. Flow herein also depends on external force (namely, external gravity), and thus it can become flow under normal gravity. In addition to gravity, liquid flow can also be from a low place to a high place by overcoming. For example, liquid can flow from a low place to a high place through extraction, compression or pressure received by it. Alternatively, liquid against its own gravity in relation to pressure can flow.

### Sample collection element with capillary channel

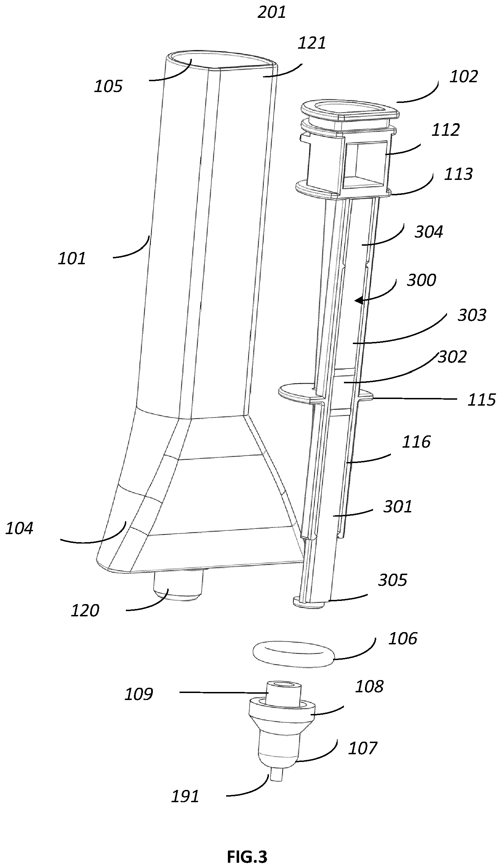

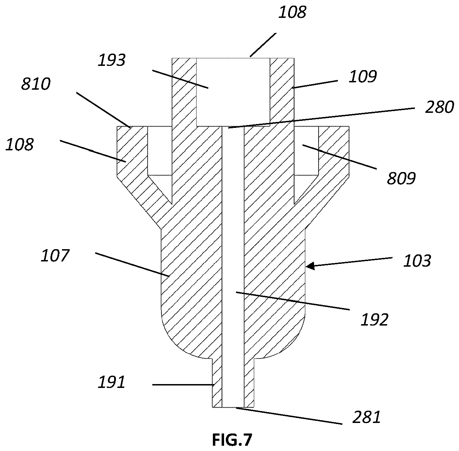

The capillary channel of the present invention is used to absorb samples, such as fluid sample, and preferably liquid samples; the liquid samples may be liquid samples or be obtained by dissolving solid samples with the solution, such as blood, saliva, urine, spinal fluid, tissue grinding fluid, drinking water, soil leaching liquor and hair grinding extract. In some embodiments, the blood may be whole blood, plasma, serum and other different forms or categories. The capillary channel herein may be a single capillary channel or a plurality of capillary channels, where each capillary channel has an inlet and an outlet, and the length of the capillary channel is defined between the outlet and the inlet. The single capillary channel of the present invention can be made of plastic, glass, metal, alloy and other materials, and can be prepared through direct injection molding or insertion of the single capillary channel into the collection element of the present invention. The capillary channel can automatically absorb liquid samples such as blood samples through the surface tension of the liquid. For example, when a fingertip blood is collected, a fingertip is punctured with a blood collection needle, and the inlet of the capillary channel contacts the blood, and a blood sample automatically flows into the capillary channel by capillary force. The capillary tube of the present invention is a single independent capillary tube and automatically absorbs the liquid samples by the capillary force thereof. Unlike the filter papers and the test strips that absorb liquid by the capillary force, the filter papers or the test strips are randomly arranged in a plurality of capillary tubes; although they also absorb the liquid by the capillary force, the liquid can be allowed to flow from one end thereof to the other end thereof. Such random arrangement causes the liquid to flow in one direction from one end thereof to the other thereof actually depending on the continuous force of the plurality of capillary tubes. In the present invention, the single capillary channel made of a same material is adopted; the liquid flows in the capillary channel by the capillary force, but can only flow in the single capillary channel and cannot automatically flow out from the capillary channel through the outlet thereof. Once the absorbed liquid samples flow into the capillaries through the inlet thereof, the liquid stays in the capillary tubes, instead of automatically flowing out of the capillary tubes. In some embodiments, the capillary tubes of the present invention have a channel with a diameter, and the diameter of the capillary tube that can generate the capillary force can be used as a specific embodiment of the present invention. In one embodiment, the capillary channel of the present invention is a capillary 192 and has an inlet 281 and an outlet 280. The capillary 192 between the outlet and the inlet has surface tension. When contacting the liquid, the inlet 281 automatically absorbs it into the capillary. The absorption volume of the liquid may be 1-50 µL, and can be defined through change in the length and diameter of the capillary, for example, 1 µL, 2 µL, 3 µL, 4 µL, 5 µL, 6 µL, 7 µL, 8 µL, 9 µL, 10 µL, 25 µL, 30 µL, 40 µL, 45 µL, and 50 µL. For example, as shown in FIG. 3 and FIG. 7, the capillary 191 is a capillary channel running through the collection element 103. The collection element includes a head portion 107 and a tail portion 108, and further includes a chamber 109 of a buffer chamber 193. The capillary channel 191 runs through the head portion and tail portion of the collection element. The inlet 281 at one end thereof extends outward from the top of the head portion 107, while the outlet 280 is in fluid communication with a buffer chamber 193. For example, the outlet 280 is directly connected with the buffer chamber 193, and the liquid flowing out of the outlet flows into the buffer chamber. In some embodiments, the volume of the buffer chamber is generally larger than the volume of the whole capillary channel. In one embodiment, the chamber 109 where the buffer chamber 193 is defined extends outward from the tail portion 108 of the collection element, such that the sealing ring can be sleeved at the periphery of the chamber 109. When the collection element 103 is manufactured, a microfluidic channel 192 can be formed in the collection element through injection molding; alternatively, a channel can be formed by plastic injection molding, and a fluid channel made of glass can be inserted into the channel, where one end of the channel protrudes outward from the top of the head portion 107 of the collection element, and the other end thereof is in communication with the buffer chamber 193. The main function of such protrusion is to allow the channel to directly contact the liquid sample, such that liquid sample can flow into the capillary through the inlet of the capillary channel. It can be understood that the capillary tube does not necessarily extend from the tail portion of the collection element, and the inlet 281 can be located at the tail portion or inside the collection element; the capillary tube can be in communication with the outside provided that the collection element has an opening. Therefore, when the tail portion of the collection element contacts the liquid sample, it is enough to allow the liquid sample to contact the inlet 281 of the capillary channel. The capillary channel may be filled with the liquid flowing into the capillary channel in all or in part, but the liquid is only preserved or stays in the capillary channel, and cannot flow out automatically of the outlet of the capillary channel. Here, it is a preferred way to provide the buffer chamber 193; alternatively, the buffer chamber 193 can be directly defaulted, the outlet 280 of the capillary channel is allowed to be directly in fluid communication with the detection chamber 105, the liquid flowing out of the capillary channel 192 directly flows into the detection chamber 105 and contacts the testing element 300 therein.

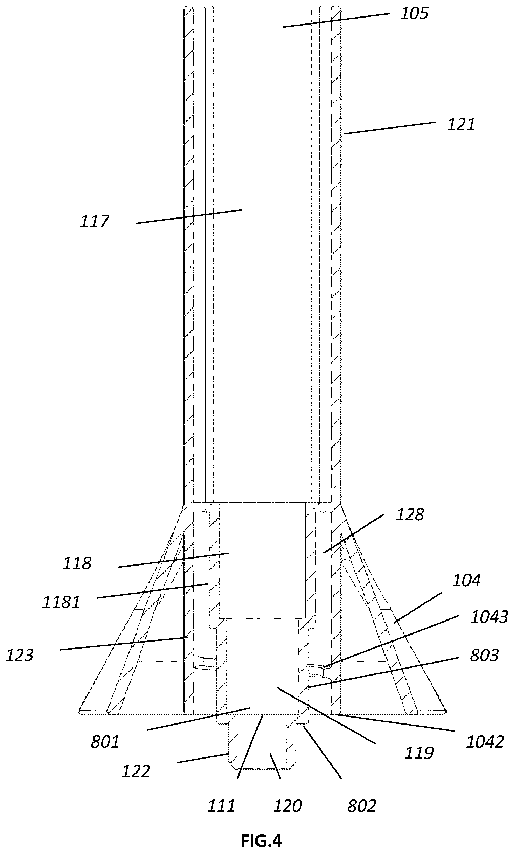

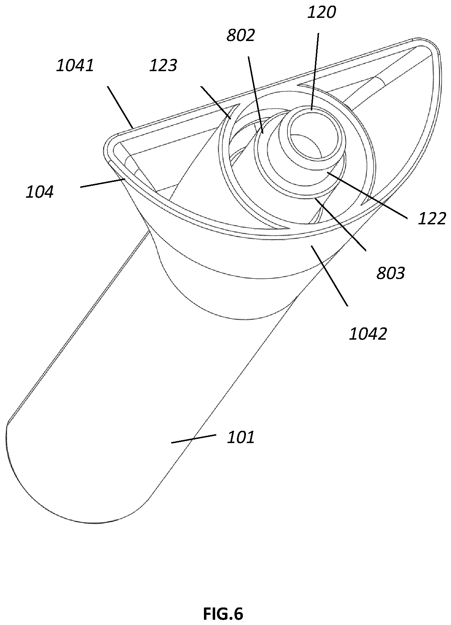

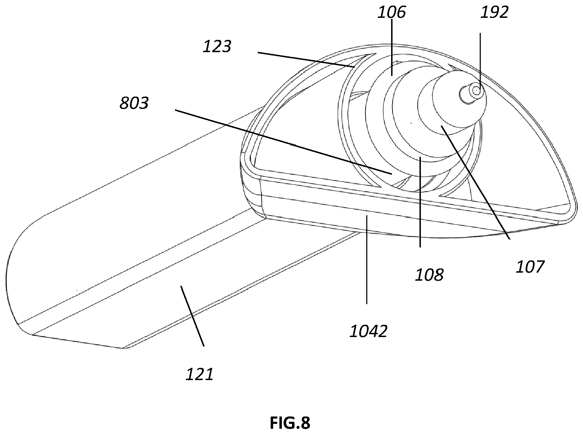

In some embodiments, the detection chamber 105 has a structure for allowing the collection element to be assembled with the detection chamber. Through the structure, the collection element 103 and the detection chamber 121 are assembled together, and the capillary tube or the capillary channel 192 on the collection element is kept in fluid communication with the detection chamber 105, and such fluid communication may be direct or indirect through the buffer chamber 193. As shown in FIG. 4 and FIG. 6, the bottom of one end of the detection chamber is provided with an open chamber 120 (which may be referred to as a "connecting chamber"), and the chamber is cylindrical and enclosed by an annular wall 122; at the position where the annular wall is proximal to the bottom 801 of the detection chamber 105, an annular wall 803 encloses the outer surface of the wall of the chamber 120, such that a step 802 is formed outside the chamber 120, and the chamber 106 extends outward from the step 802; apparently, the diameter of the chamber 119 enclosed by the wall 803 is greater than that of the chamber 120 enclosed by the wall 122, such that a step 802 is formed at the transition position of the two chambers. Of course, the walls 122 of the chamber 120 have a specific thickness, for example, 1-2 mm thick or above. In addition, the collection element has an annular chamber 804 at the head portion thereof, and the buffer chamber 193 is also enclosed by the wall 109. There is a distance between the wall 109 of the buffer chamber and the wall 108 of the annular chamber 804 at the head portion thereof to form an annular spacing area 809, and the width of the annular spacing area 809 is slightly equal to or less than the thickness of the annular wall 122 on the detection chamber. Therefore, during the assembly, the open chamber 120 of the detection chamber is allowed to be inserted to the annular spacing area 809 on the collection element 103, such that the collection element and the detection chamber are assembled together through the chamber 120 of the detection chamber, and the chamber 109 with the buffer chamber 193 is located in the open chamber 120 of the chamber 109. The assembling structure is as shown in FIG. 8. The sealing ring is sleeved outside the cylindrical chamber 109, and the sealing ring is able to lean against the edge 810 of the wall 108 of the annular chamber 804, and the diameter of the sealing ring 106 is larger than the thickness of the edge 810, such that the sealing ring is fixed by the edge 810 of the wall 108 of the chamber 804 and the annular step 802. As the sealing ring has elasticity, the elastic sealing ring is fixed between the edge 810 of the wall 108 of the chamber 804 and the annular step 802, and the sealing ring 106 protrudes slightly outward from the annular step (FIG. 8), thereby facilitating the sampling element to be inserted into a chamber. The elastic sealing ring can be compressed transversely to constitute a seal, and the following will give a detailed description. In this case, the buffer chamber 109 is located in the chamber 106 and under the bottom of the detection chamber. It can be understood that the capillary channel can be circular or any shape, such as square, rhombic, and oval, and any capillary channel that can automatically absorb liquid through the surface tension of the liquid can be regarded as a specific embodiment of the present invention.

### Carrier of testing element

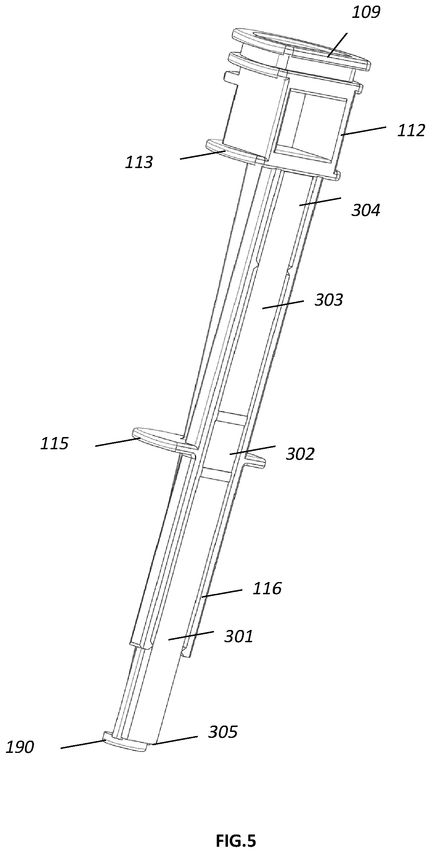

In addition to the above test strip or lateral flow test strip 300 itself being used to contact a liquid sample to test presence or absence of analytes therein, if only the testing element is available, the testing element can be erected directly in the detection chamber 105, and a sample application area 301 of the testing element is located on the bottom 801 of the detection chamber and proximal to the outlet 280 of the capillary channel, such that the liquid flowing out of the capillary channel can flow into a sample reagent area of the testing element to participate in the liquid flow of the testing element, thereby assaying and testing the analytes. In some preferred embodiments, the testing element can also be provided on some carriers, as shown in FIG. 5. For example, some carriers 102 are provided with grooves 116, and the testing element 300 is located in the grooves. The carrier 102 includes an element fixed onto the detection chamber, as shown in FIG. 4. The element fits with the inner chamber of the detection chamber, such that the carrier can be inserted into the detection chamber 105. With the carrier being inserted into the detection chamber 105, the carrier having the testing element is located in the detection chamber, as shown in FIG. 6. The detection chamber 105 is a hollow chamber; one end thereof fits with one end 109 of the carrier; and the other end 190 thereof is located in the chamber, also serves as one end 305 of the sample application area 301 of the testing element 300, and is located at the bottom 801 of the other end of the detection chamber. The other end of the detection chamber has a location for contacting the carrier, such that the carrier is provided in the detection chamber. The testing element is further provided with the label area 302 downstream of the sample application area 301; and a testing area 303 is further provided downstream of the label area. Biochemical reagents are treated on the testing area, and subjected to immune or chemical reactions thereon, which displays reaction results. Through the reaction results, presence or absence of analytes in test samples or the number of the analytes can be judged. The carrier is fixed in the chamber in such a way that annular edges 115, 113 on the carrier is allowed to contact the inner walls of the detection chamber 105 to fix the carrier in the chamber. To allow the carrier to be inserted into the chamber in a fixed direction, one end 109 of the carrier has a shape fitting with one end of the chamber; generally, the shape is directional instead of being cylindrical. For example, the detection chamber is enclosed by a circular wall 101 and a planar wall 121; therefore, an irregular circle fitting with a plane is formed on one end of the detection chamber 105, and the carrier also has a shape fitting with it. For example, the groove 116 in the carrier directly faces the planar wall 121, and the annular elements 115, 113 for fixing the carrier are in direct contact with the inner walls of the circular walls 101 of the detection chamber, thereby allowing the carrier to be inserted into the detection chamber 105 in a fixed direction. The groove is allowed to directly face the planar wall 121, such that the testing area 303 of the testing element 300 also directly faces the planar wall 121 and the test results of the testing area 303 on the testing element 300 can be read through the planar wall 121. Reading herein generally means that the area on the planar wall 121 corresponding to the testing area is transparent and the rest results can be read directly by naked eyes or by reading equipment.

### Test device

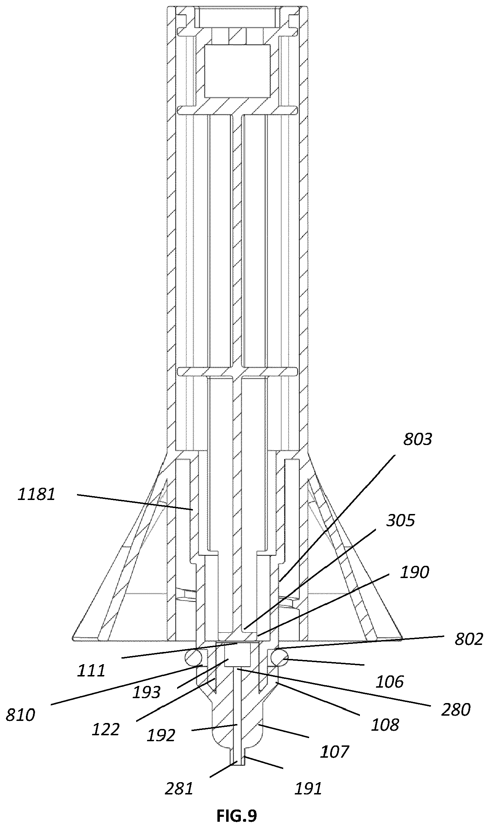

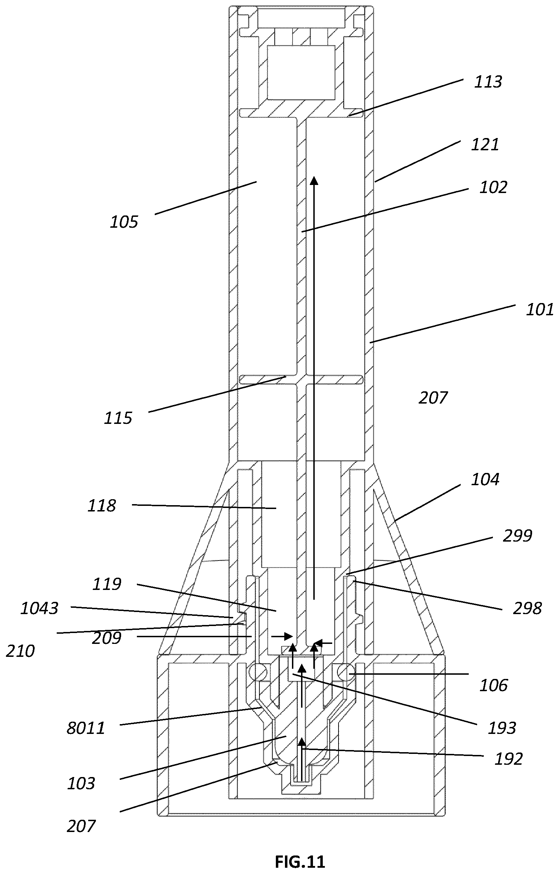

Test device refers to a device for testing presence or absence of an analyte in a sample. The test device herein can simply include a detection chamber 105 and a testing element 300 therein, so it can be called a test device. For example, the test device includes the detection chamber 105, where the detection chamber 105 includes the testing element 300 or the carrier 102 including the testing element 300. In some embodiments, the detection chamber 105 has a liquid inlet 111, and the liquid sample flows into the bottom 801 of the detection chamber 105 through the liquid inlet 111 to contact the testing element 304. In some embodiments, the sample application area of the testing element is proximal to the liquid inlet 111, such that the liquid flows into the detection chamber through the liquid inlet 111 and can contact the sample application area, and the liquid sample flows into the testing area along the sample application area, thereby assaying and testing the analytes. As shown in FIG. 9 and FIG. 11, the liquid inlet 111 is provided near the bottom 801 of the detection chamber 105. In some embodiments, the liquid inlet 111 is in direct communication with the buffer chamber 193, that is, the opening 198 of the buffer chamber 193 is in direct communication with the bottom 801 of the detection chamber without any partition between them; the bottom end 190 of the carrier is suspended in the detection chamber; and the buffer chamber 193 is located between the detection chamber and the capillary outlet. As described above, one function of the buffer chamber 193 is to reduce the flow velocity of the liquid sample. As the flow velocity of the liquid flowing out of the capillary tube is fast, the flow velocity of the liquid having the fast flow velocity slows down in the buffer chamber. This is because the volume of the buffer chamber is relatively large, and generally larger than the volume of the capillary tube, the volume of the whole capillary tube, or the volume of the liquid sample preserved in the capillary tube, the flow velocity of the liquid slows down in the buffer chamber, and then flows into the detection chamber at a relatively slow flow velocity after flowing into the bottom of the detection chamber. The other function thereof is that when the treatment liquid flows into the capillary tube (in a case that detection starts to be conducted, the liquid sample is automatically absorbed through the inlet of the existing capillary tube), the treatment liquid is generally located near the inlet of the capillary tube and the liquid sample is located near the outlet of the capillary tube. When the treatment liquid facilitates the liquid sample to move in the capillary tube, the liquid sample and the treatment liquid flow into the buffer chamber sequentially, such that the treatment liquid and the liquid sample are well mixed in the buffer chamber, thereby achieving the purpose of treating the liquid sample with the treatment liquid. Then, the prepared mixture flows into the bottom of the detection chamber, and contacts the sample application area of the testing element, thereby completing the test of the analyte in the liquid sample.

In some embodiments, a partition plate 199 can be provided at the inlet 111; the bottom end 190 of the carrier is in contact with the partition plate 199; and the partition plate is provided with holes (omitted in the figure), such that the liquid flowing out of the buffer chamber 193 flows into the bottom 801 of the detection chamber 105 through the holes in the partition plate. With the provision of the partition plate, the buffer chamber can be omitted; and the outlet of the capillary tube is in direct communication with the detection chamber through the partition plate. Of course, it is also a preferred solution to provide the buffer chamber. In some embodiments, the holes in the partition plate and the outlet of the capillary channel are not located in a straight line; therefore, the liquid at the outlet of the capillary tube is allowed to contact the location of the partition plate without the holes to slow down the flow velocity thereof, instead of directly flowing into the detection chamber through the holes; then, the liquid flows into the bottom of the detection chamber and is gathered at the bottom, such that the sample application area of the testing element is in contact with the liquid to perform a chromatographic assay. One function of the partition plate is to slow down the flow velocity of the liquid in the buffer chamber flowing into the detection chamber. Generally, when the sampling element is fully inserted into the chamber, the liquid in the capillary channel quickly flows into the buffer chamber to be mixed, and the liquid in the buffer chamber 193 quickly flows into the bottom 801 of the detection chamber 105 under external pressure. If the flow velocity is too fast, the liquid can be sprayed into the detection chamber, and splashes to the label area in advance and moistens it in advance. However, in a case that the label area is moistened in advance, the flow velocity of the liquid to moisten the sample application area of the testing element slows down, the testing results of the testing element are inaccurate because the lateral flow test strip is used to test the analyte in the liquid sample. It is desired that the liquid sequentially flows into the sample application area 301, the label area 302, the testing area 303, and the water absorption area 304, so the testing results are accurate. The partition plate is provided at the liquid inlet 111 to reduce the impact of the liquid flowing from the buffer chamber to the detection chamber, which allows the liquid to flow into the detection chamber slowly. In some embodiments, if the outlet 280 of the capillary channel is in direct communication with the bottom 801 of the detection chamber in absence of the buffer chamber, the flow velocity of the liquid flowing out of the capillary channel is relatively fast, and in this case, one partition plate also needs to be provided in the detection chamber and above the outlet 280 of the capillary channel. The partition plate is preferably located at the bottom 801 of the detection chamber 801, and of course, can also be located below the label area of the testing element, such that the liquid flows into the detection chamber, and firstly contacts the sample application area instead of the label area.

In some embodiments, the detection chamber 105 is divided into three sections of the chamber with different cross-sectional areas, and the cross-sectional areas thereof are reduced once from one end to the bottom 801, where the three sections are a first section 117, a second section 118 and a third section 119, respectively; and the carrier for carrying the testing element 300 is provided in the whole detection chamber 105. A wall 123 is also encircled outside the second section of the detection chamber and the third section thereof, and an encircling raised edge 1043 is provided inside the encircled wall and similar to a raised apron. In addition, a long and narrow channel 128 is present between the encircling wall 123 and the walls 1181, 1191 of the second section 118 and third section 119 of the detection chamber, and is used to allow the bottleneck of the receiving chamber to enter the long and narrow channel. This will be described in detail later. In addition, the diameter of the second section of the detection chamber is larger than the diameter of the third section thereof, such that a step 299 is formed during the transition thereof and is in contact with the edge 298 of the opening 292 of the receiving chamber, thereby limiting the insertion depth of the collection element; actually, such depth is to allow the inlet of the capillary channel to approach the bottom of the lower chamber 291 of the receiving chamber, and of course, can be freely set, provided that the inlet of the capillary channel is not sealed by contacting the bottom. In this case, liquid or air can be allowed to smoothly enter into the capillary channel, thereby facilitating the liquid sample to flow out of the capillary channel and flow into the detection chamber.

### Receiving chamber

In some preferred embodiments, the present invention further provides a chamber including a treatment liquid; the treatment liquid chamber and the detection chamber are movable relative to each other, allowing the sampling element to be inserted into the chamber, which facilitates a liquid sample retained in a capillary tube to flow out of the capillary tube through the outlet thereof and flow into the detection chamber. In some embodiments, the receiving chamber allows the whole collection element to enter the chamber. With the collection element entering the chamber and substantially occupying the shape of the chamber as a whole, a part of the treatment liquid in the chamber flows into the capillary tube and allows it to continue to flow therein, which facilitates the liquid sample to flow out of the capillary tube and flow into the detection chamber to contact the testing element therein. In some embodiments, the collection element is inserted into the chamber and seals the opening of the chamber; a sealing space is formed in the chamber; the volume of the sealing space is compressed, and the air pressure thereof increases; the increased air pressure causes the gas (for example, air) and/or the treatment liquid in a sealing chamber to enter the capillary channel through the inlet thereof, which facilitates the liquid sample therein to flow out of the capillary channel through the outlet thereof; and an efflux may be only the liquid sample, or may be a mixture of the liquid sample and the treatment liquid.

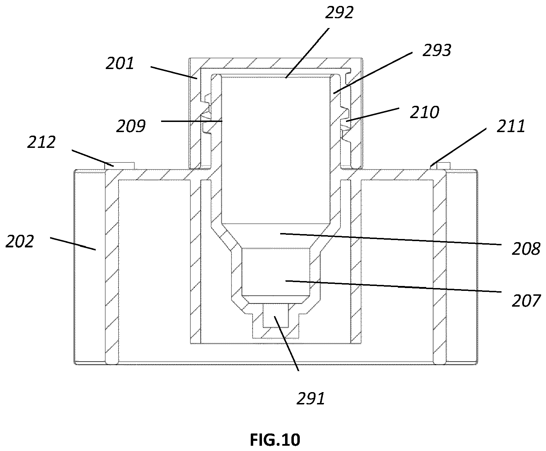

In some embodiments, the shape of the receiving chamber resembles the shape of a bottle, as shown in FIG. 10. A chamber 208 with an opening 292 is provided in the bottle, where the opening is enclosed by walls 209 of the bottleneck; and the shape of the chamber below the bottleneck fits with that of the collection element 103, which is similar to the inverted design of the collection element. The chamber is also divided into an upper chamber 208, an intermediate chamber 207, and a lower chamber 291; the shape of the upper chamber 208 fits with a tail portion 108 of the collection element; the intermediate chamber 207 fits with a head portion 107; and the lower chamber 291 fits with the top of the tail portion of the collection element. When the collection element 107 enters the chamber as a whole, it substantially occupies the whole chamber, providing at most a small gap between the collection element and the chamber. "Matching" herein means that when the collection element enters the receiving chamber, the inner space of the chamber is almost filled with the collection element substantially, and the collection element can occupy 99%, 95%, 90%, 85%, 80%, 75%, 65%, 60% and 50% of the space. When the collection element occupies 85-99% of the space or 60-99% of the space, and the collection element enters the receiving chamber, it is not necessary to form the sealing space therein. This is because the collection element entering the receiving chamber occupies almost most of the space of the receiving chamber. In this case, if the treatment liquid is in advance stored in the receiving chamber and when the collection element occupies the whole space of the receiving chamber, the treatment liquid is bound to disperse around the collection element. There is a very small gap 8011 between the outer surface of the collection element and the inner surface of the receiving chamber, and the liquid is almost difficult to quickly flow out of the gap. In this case, the inlet of the capillary channel is in contact with the treatment liquid. When the detection chamber is in communication with external atmosphere, the collection element actually exerts pressure to the liquid, and the pressure causes the treatment liquid to flow into the capillary channel, thereby facilitating the liquid sample stored in the capillary channel to flow out of the capillary channel and flow into the detection chamber or sequentially flow into the buffer chamber and the detection chamber. When the collection element occupies 90-99% of the space of the receiving chamber, or when the treatment liquid is difficult to quickly flow out of the gap formed between the outer surface of the collection element and the inner surface of the receiving chamber, it is unnecessary to form a sealing structure in the receiving chamber.

Of course, in some preferred embodiments, the collection element is inserted from the opening 292 of the receiving chamber and the elastic sealing ring 106 on the collection element is in contact with the inner wall 209 of the opening, thereby sealing the opening 292. Such sealing may be hermetic sealing or liquid sealing. In case of hermetic sealing, the elastic sealing ring 106 moves downward together with the collection element entering the receiving chamber, and the volume of the sealing space in the receiving chamber is reduced. If the receiving chamber only has air instead of the treatment liquid, a par of air enters the capillary tube with the increase of pressure, and the air entering the capillary tube facilitates the liquid sample automatically absorbed by the capillary tube to flow out of the capillary tube and the air entering the capillary tube facilitates flow into the detection chamber to contact the testing element therein. If the receiving chamber includes the treatment liquid therein in advance, the elastic sealing ring 106 moves downward together with the collection element entering the receiving chamber in case of hermetic sealing. If the treatment liquid further includes gas (air), the gas enters the capillary channel, facilitating the liquid sample in the capillary channel to flow into the buffer chamber. If the air is exhausted, the treatment liquid sequentially enters the capillary channel and the buffer channel to be mixed with the liquid sample, and then enters the detection chamber 105 to contact the testing element. In case of liquid sealing, the receiving chamber preferably needs to include the treatment liquid therein in advance, and the opening 292 of the receiving chamber is liquid-sealed by the elastic sealing ring 106. In this case, the elastic sealing ring 292 moves downward with the collection element; if there is excess gas in the sealing space, the gas is discharged through the space between the elastic sealing ring 292 and the opening wall 209, but the liquid cannot be discharged in such a way. The treatment liquid only can flow into the capillary channel, facilitating the liquid sample in the capillary channel to flow into the buffer chamber to allow the treatment liquid and the liquid sample to be mixed. It can be understood that the capillary channel sometimes does not automatically absorb the liquid sample. When solid samples or powder samples are taken, the inlet of the capillary channel is allowed to be inserted into the solid samples or powder samples, and powder adheres at or around the inlet of the capillary channel. Then, the treatment liquid is allowed to flow into the capillary channel through the above operation, thereby causing the powder samples to enter the capillary channel and flow out of the outlet thereof together. In this case, the treatment liquid plays the role of dissolving the solid samples, and then flows into the detection chamber to contact the testing element for testing or detection. The powder can be drugs or any other solid samples.

When the test device is generally inserted vertically into the receiving chamber (FIG. 11), a sufficient amount of the treatment liquid flows into the capillary channel and into the buffer chamber 193 due to the continuously reduced volume of the sealing chamber, and the mixture flows into the detection chamber when the buffer chamber is fully filled; the detection chamber includes a specific level height of the liquid; and the liquid contacts the sample application area of the testing element to complete the test of the analyte. The liquid sample may be in unit of µL, for example, 1-50 µL or 0.5 µL. However, with the treatment liquid being sufficiently provided, the total volume of the treatment liquid that dissolves the liquid sample is greater than the volume of the buffer chamber 193 and approximately 500-2 mL, thereby ensuring presence of the liquid or the mixture (obtained by mixing the treatment liquid with the liquid sample) to flow into the detection chamber.

The sampling element 103 is allowed to be inserted into the receiving chamber, and the treatment liquid in the receiving chamber is allowed to flow into the capillary channel, such that the liquid sample in the capillary channel flows into the detection chamber or the treatment liquid and the liquid sample therein together flow into the detection chamber. Therefore, detection can be performed simply and quickly, and completed through direct operation, without any specialized training. In some embodiments, the sampling element 103 is locked with the solution chamber 200 after inserted into the chamber. In some embodiments, a part of the sampling element is inserted into an accommodating chamber in which the sealing chamber is formed; the sampling element continues to move, allowing the sealing chamber to be reduced in volume; if gas is present in the sealing chamber, the volume of the gas is compressed, the pressure therein increases, and the increased pressure causes the liquid sample in the capillary channel to flow into the detection chamber.

There may be many options to implement the locking structure. When the elastic sealing ring 106 is used to seal the opening 292 of the receiving chamber, the sampling element 103 and the receiving chamber are allowed to form a fixed integrated structure when detection is completed, and the sampling element 103 and the detection chamber 101 are integrally connected. Therefore, the receiving chamber and the whole test device are allowed to form a locking structure; after the testing results are read, the sampling element can be directly discharged, which reduces the environmental pollution caused by scattered discarding. Another way to implement the locking structure is that a groove 210 is provided in the outer surface of the wall 209 enclosing the bottleneck. One function of the groove is to prevent the treatment liquid from leakage when a cover 201 is used to seal the opening 209 of the receiving chamber. During detection, the cover 201 is removed and the collection element 103 is allowed to be inserted into the receiving chamber 202, which actually allows the whole collection element to enter the receiving chamber. In the test device, a wall 123 is also encircled outside the second section of the detection chamber and the third section thereof, and an encircling raised edge 1043 is provided inside the encircled wall and similar to a raised apron. In addition, a long and narrow channel 128 is present between the encircling wall 123 and the walls 1181, 1191 of the second section 118 and third section 119 of the detection chamber, and is used to allow the bottleneck of the receiving chamber or the wall 209 enclosing the opening to enter the long and narrow channel, and the raised apron 1043 in the channel enters the groove, thereby implementing the locking structure of the test device and the receiving chamber. Of course, there is a third way, that is, a cover structure 104 is formed around the collection element of the detection chamber and has a wall 1042 with a lock thereon. When the collection element 103 is inserted into the receiving chamber, the lock is sequentially provided inside the wall of the cover structure and forms a locking structure with a lock 211, 212 outside the receiving chamber, such that the test device and the receiving chamber are integrally connected and inseparable. Any of the three ways may be used, or all the three ways are used at the same time. It can be understood that any locking structure to realize the inseparability of objects connected together can be used in the present invention and is a specific embodiment of the present invention.

The operation of the device of the present invention is described below with reference to FIG. 11. After assembled, the test device includes the detection chamber 105; the carrier 102 is provided in the detection chamber and includes the testing element 300; the carrier is located in the detection chamber and the sample application area of the testing element is located at the bottom 801 of the detection chamber; the end portion of the sample application area is located on the end portion 190 of the carrier; and the end portion 190 is located at the bottom of the detection chamber. The buffer chamber 193 is located under the bottom of the detection chamber. Structurally, the buffer chamber is a hollow tube 109 on the collection element. When the tube is inserted into the connecting chamber 120 of the detection chamber, the buffer chamber 193 is formed. The collection element includes the capillary channel 192l, where an inlet 281 thereof is exposed, an inlet 280 thereof is in communication with the buffer chamber, and the inlet 111 of the detection chamber is located in the buffer chamber. A wall 123 is also encircled outside the second section of the detection chamber and the third section thereof, and an encircling raised edge 1043 is provided inside the encircled wall and similar to a raised apron. In addition, a long and narrow channel 128 is present between the encircling wall 123 and the walls 1181, 1191 of the second section 118 and third section 119 of the detection chamber. The cover structure 104 is formed around the collection element of the detection chamber and has a wall 1042 with a lock thereon. The receiving chamber is a bottle, where the bottle includes a bottleneck 292 and a wall 209 enclosing the bottleneck; the outer surface of the wall is provided with a groove 210; and the inner surface thereof is a smooth surface. A space is provided inside the bottle, where the overall shape of the space fits with the shape of the collection element 103; one space 291 is provided at the bottom of the bottle and used as the final location of the capillary inlet at the end portion of the collection element; the bottle is filled with the treatment liquid during delivery; and the opening 292 is sealed with a bottle cover 201.

In case of test, the inlet 281 of the capillary tube 192 is allowed to contact the liquid sample, and the liquid sample automatically flows into the capillary tube by capillary force and is stored in the capillary tube. Then, the bottle cover including the treatment liquid is removed and the collection element is inserted into the chamber of the receiving chamber; and the opening is sealed with the elastic sealing ring; in this case, the treatment liquid flows into the capillary channel, facilitating the liquid to flow out of the capillary channel and flow into the buffer chamber 193; the treatment liquid and the liquid sample are allowed to be mixed in the buffer chamber; with an excessive amount of the treatment liquid flowing into the capillary tube and the buffer chamber, after the buffer chamber is filled with the mixture, the mixture flows into the detection chamber and contacts the sample application area of the testing element to complete the test. The liquid sample herein may be blood, such as fingertip blood, or may be a powder-like solid sample. During collection, the capillary inlet is allowed to contact the solid sample or be inserted into the solid sample such that the powder is allowed to adhere to the inlet, and the treatment liquid flows into the capillary channel, dissolves the powder, and then flow into the detection chamber.

All embodiments as below are also part of the Invention.
1. A device for testing an analyte in a liquid sample, comprising:
   a detection chamber, wherein the detection chamber is configured to receive a testing element, and the testing element is configured to test the analyte in the liquid sample; and
   a sampling element, wherein the sampling element is configured to absorb the liquid sample wherein the sampling element comprises a capillary channel, and the capillary channel is capable to automatically absorb the liquid sample and is in fluid communication with the detection chamber.
2. The device according to clause 1, wherein the capillary channel is provided in one or plurality, and each capillary channel is capable to automatically absorb the liquid sample.
3. The device according to one of clauses 1-2, wherein after the capillary channel absorbs the liquid sample, the liquid sample is stored in the capillary channel, but is incapable to flow into the detection chamber.
4. The device according to one of clauses 1-3, wherein the capillary channel comprises an inlet and an outlet; the liquid sample flows into the capillary channel through the inlet; and the outlet is in fluid communication with the detection chamber.
5. The device according to one of clauses 1-4, wherein the sampling element comprises an insertion end for being inserted into a chamber, and the inlet of the capillary channel is provided on the insertion end.
6. The device according to one of clauses 1-5, wherein the sampling element is used to be inserted into the chamber as a whole, such that pressure at the inlet of the capillary channel increases when the sampling element is inserted into the chamber, and the increased pressure causes the liquid sample in the capillary channel to flow out of the outlet thereof.
7. The device according to one of clauses 1-6, wherein the chamber comprises a treatment liquid therein; when the sampling element is inserted into the chamber, the increased pressure causes the treatment liquid to flow into the capillary channel, whereby facilitating the liquid sample in the capillary channel to flow out of the outlet thereof.
8. The device according to one of clauses 1-7, wherein an interior shape of the chamber fits with a shape of the insertion end, such that there is only a slit between an outer surface of the insertion end and an inner surface of the chamber when the insertion end is inserted into the chamber, and the slit does not allow the liquid sample to flow out of the chamber.
9. The device according to one of clauses 1-8, wherein when the insertion end is inserted into the chamber, the insertion end is combined with inner walls of the chamber to form a sealing space in the chamber; with the insertion end being further inserted into the chamber, air in the sealing space is compressed, such that air pressure in the sealing space increases, and the increased air pressure causes a part of gas or the treatment liquid to flow into the capillary channel.
10. The device according to one of clauses 1-9, wherein the chamber comprises an opening, and the insertion end seals the opening of the chamber when being inserted into the chamber.
11. The device according to clause 10, wherein the sealing is hermetic sealing or liquid sealing.
12. The device according to clause 11, wherein a buffer chamber is further provided between the outlet of the capillary channel and the detection chamber, and the buffer chamber allows the liquid sample flowing out of the capillary channel through the outlet thereof to be mixed with the treatment liquid.
13. The device according to clause 10, wherein when the insertion end is inserted into the chamber, the insertion end is locked with the chamber and is incapable to withdraw from the chamber.
14. The device according to clause 1, wherein the liquid sample is a blood sample.
15. A device for testing an analyte in a liquid sample, comprising:
   a detection chamber, wherein the detection chamber comprises a testing element, and the testing element is configured to test the analyte in the liquid sample;
   a sampling element, wherein the sampling element comprises a capillary channel, the capillary channel is configured to automatically absorb a blood sample and allow the blood sample to be stored in the capillary channel, and the capillary channel is in fluid communication with the detection chamber; and
   a solution chamber, configured to accommodate a treatment liquid for treating the liquid sample;
   wherein when a part of the sampling element is inserted into the solution chamber, the sampling element forms a hermetic sealing chamber in the solution chamber, and the chamber comprises the treatment liquid therein; when the sampling element continues to be inserted, the sampling element causes pressure in the hermetic sealing chamber to increase, and the increased pressure causes the treatment liquid to flow into the capillary channel, whereby causing the liquid sample to flow out of the capillary channel and flow into the detection chamber.
16. A method for testing an analyte in a liquid sample, comprising:
   providing a device, wherein the device comprises a detection chamber; the detection chamber is configured to receive a testing element, and the testing element is configured to test the analyte in the liquid sample; and a sampling element, wherein the sampling element is configured to automatically collect the liquid sample, and the sampling element comprises a capillary channel;
   allowing the capillary channel of the sampling element to automatically absorb the liquid sample; then inserting the sampling element into a solution chamber comprising a treatment liquid such that the sampling element causes the treatment liquid in the solution chamber to flow into the capillary channel, whereby allowing the liquid sample in the capillary channel to flow into the detection chamber.
17. The method according to clause 16, wherein the treatment liquid is allowed to flow into the capillary channel to contact the liquid sample.
18. The method according to clause 17, wherein after the capillary channel is allowed to automatically absorb the liquid sample, the liquid sample is allowed to be stored in the capillary channel, but is incapable to flow out of the capillary channel.
19. The method according to clause 18, wherein the sampling element is allowed to form a hermetic sealing chamber or a liquid sealing chamber in the solution chamber comprising a treatment liquid, and the sealing chamber comprises liquid or gas therein; when the sampling element continues to move in the chamber, pressure in the sealing chamber is allowed to increase, and the increased pressure causes the gas or the treatment liquid in the sealing chamber to flow into the capillary channel, whereby facilitating the liquid sample to flow out of the capillary channel and flow into the detection chamber.
20. The method according to clause 19, wherein the capillary channel comprises an inlet for absorbing the liquid sample and an outlet for allowing the liquid sample to flow out of the capillary channel; the outlet is in fluid communication with the detection chamber; a buffer chamber is provided between the outlet of the capillary channel and the detection chamber; and the buffer chamber allows the liquid sample flowing out of the capillary channel to be mixed with the treatment liquid.
21. The method according to clause 17, wherein the liquid sample is a blood sample.
22. The method according to clause 22, wherein the analyte is virus, bacterium, fungus, and other substances.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where such restriction is not specifically described here. For example, the terms "comprising", "essentially consisting of" and "consisting of" in each example herein may be replaced by the rest 2 terms. The so-called "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. A person skilled in the art can make some modifications and changes according to the essence of the description of the present invention. These modifications and changes are also considered to fall within the scope of the present invention and the scope limited by independent claims and dependent claims.

## Claims

1. A device for testing an analyte in a liquid sample, comprising:
a detection chamber, wherein the detection chamber is configured to receive a testing element, and the testing element is configured to test the analyte in the liquid sample; and
a sampling element, wherein the sampling element is configured to absorb the liquid sample wherein the sampling element comprises a capillary channel, and the capillary channel is capable to automatically absorb the liquid sample and is in fluid communication with the detection chamber.

2. The device according to claim 1, wherein the capillary channel is provided in one or plurality, and each capillary channel is capable to automatically absorb the liquid sample.

3. The device according to one of claims 1-2, wherein after the capillary channel absorbs the liquid sample, the liquid sample is stored in the capillary channel, but is incapable to flow into the detection chamber.

4. The device according to one of claims 1-3, wherein the capillary channel comprises an inlet and an outlet; the liquid sample flows into the capillary channel through the inlet; and the outlet is in fluid communication with the detection chamber.

5. The device according to one of claims 1-4, wherein the sampling element comprises an insertion end for being inserted into a chamber, and the inlet of the capillary channel is provided on the insertion end.

6. The device according to claim 5, wherein the sampling element is used to be inserted into the chamber as a whole, such that pressure at the inlet of the capillary channel increases when the sampling element is inserted into the chamber, and the increased pressure causes the liquid sample in the capillary channel to flow out of the outlet thereof.

7. The device according to one of claims 5-6, wherein the chamber comprises a treatment liquid therein; when the sampling element is inserted into the chamber, the increased pressure causes the treatment liquid to flow into the capillary channel, whereby facilitating the liquid sample in the capillary channel to flow out of the outlet thereof.

8. The device according to one of claims 5-7, wherein an interior shape of the chamber fits with a shape of the insertion end, such that there is only a slit between an outer surface of the insertion end and an inner surface of the chamber when the insertion end is inserted into the chamber, and the slit does not allow the liquid sample to flow out of the chamber.

9. The device according to claim 8, wherein when the insertion end is inserted into the chamber, the insertion end is combined with inner walls of the chamber to form a sealing space in the chamber; with the insertion end being further inserted into the chamber, air in the sealing space is compressed, such that air pressure in the sealing space increases, and the increased air pressure causes a part of gas or the treatment liquid to flow into the capillary channel.

10. The device according to claim 10, wherein the chamber comprises an opening, and the insertion end seals the opening of the chamber when being inserted into the chamber.

11. The device according to claim 11, wherein the sealing is hermetic sealing or liquid sealing.

12. The device according to claim 11, wherein a buffer chamber is further provided between the outlet of the capillary channel and the detection chamber, and the buffer chamber allows the liquid sample flowing out of the capillary channel through the outlet thereof to be mixed with the treatment liquid.

13. The device according to claim 10, wherein when the insertion end is inserted into the chamber, the insertion end is locked with the chamber and is incapable to withdraw from the chamber.

14. A device for testing an analyte in a liquid sample, comprising:
a detection chamber, wherein the detection chamber comprises a testing element, and the testing element is configured to test the analyte in the liquid sample;
a sampling element, wherein the sampling element comprises a capillary channel, the capillary channel is configured to automatically absorb a blood sample and allow the blood sample to be stored in the capillary channel, and the capillary channel is in fluid communication with the detection chamber; and
a solution chamber, configured to accommodate a treatment liquid for treating the liquid sample;
wherein when a part of the sampling element is inserted into the solution chamber, the sampling element forms a hermetic sealing chamber in the solution chamber, and the chamber comprises the treatment liquid therein; when the sampling element continues to be inserted, the sampling element causes pressure in the hermetic sealing chamber to increase, and the increased pressure causes the treatment liquid to flow into the capillary channel, whereby causing the liquid sample to flow out of the capillary channel and flow into the detection chamber.

15. A method for testing an analyte in a liquid sample, comprising:
providing a device, wherein the device comprises a detection chamber; the detection chamber is configured to receive a testing element, and the testing element is configured to test the analyte in the liquid sample; and a sampling element, wherein the sampling element is configured to automatically collect the liquid sample, and the sampling element comprises a capillary channel;
allowing the capillary channel of the sampling element to automatically absorb the liquid sample; then inserting the sampling element into a solution chamber comprising a treatment liquid such that the sampling element causes the treatment liquid in the solution chamber to flow into the capillary channel, whereby allowing the liquid sample in the capillary channel to flow into the detection chamber.
